# FASCICULE DE BREVET EUROPEEN

(11) **EP 3 177 172 B1**
(45) Date de publication et mention de la délivrance du brevet: **06.10.2021**
(21) Numéro de dépôt: 15759876.4
(22) Date de dépôt: 30.07.2015
(51) Int. Cl.: A45D 34/00, A45D 34/02, A45D 40/00

(54) **SYSTÈME POUR TESTER UN PARFUM**
SYSTEM ZUM TESTEN EINES PARFUMS
SYSTEM FOR TESTING A PERFUME

(30) Priorité: 06.08.2014 FR 1457645
(43) Date de publication de la demande: 14.06.2017
(73) Titulaire: Louis Vuitton Malletier, 75001 Paris (FR)
(72) Inventeur: DUPONT, Céline, 92700 Colombes (FR); SEURAT, Aurélie, 92700 Levallois-perret (FR); GORY, Bénédicte, 75019 Paris (FR)
(74) Mandataire: Plasseraud IP
(86) Numéro de dépôt international: PCT/FR2015/052112
(87) Numéro de publication internationale: WO 2016/020605

(56) Documents cités:
- EP-A1- 1 172 305
- EP-A1- 1 243 199
- FR-A1- 2 987 761
- US-A- 5 031 764
- None

## Description

### -- DOMAINE DE L'INVENTION --

La présente invention concerne les systèmes pour tester des parfums.

Plus particulièrement, l'invention concerne un système pour tester un parfum comprenant :
- un boîtier comportant une ouverture ;
- un organe de test adapté pour être au moins partiellement inséré à l'intérieur du boîtier à travers l'ouverture, l'organe de test étant réalisé au moins partiellement à partir d'un matériau adapté pour s'imprégner du parfum ; et
- un dispositif distributeur de parfum disposé à l'intérieur du boîtier et adapté pour distribuer le parfum sur l'organe de test lorsque l'organe de test est inséré à l'intérieur du boîtier.

### -- ARRIERE-PLAN DE L'INVENTION --

On connaît du document EP 1 172 305 A1 un système de ce type dans lequel le dispositif distributeur de parfum comprend un récipient muni d'une pompe et d'une buse pour pulvériser le parfum sur l'organe de test.

Cependant, ce système met en œuvre un mécanisme complexe faisant intervenir une multitude de pièces coopérant les unes avec les autres.

Un système similaire et présentant les mêmes inconvénients est décrit dans le document FR 2 987 761 A1. Ce système requiert en outre la présence de composants électriques tels qu'un moteur, une alimentation électrique ainsi qu'un circuit électronique complet de détection comprenant notamment un microprocesseur et un émetteur-récepteur infra-rouge pour la détection de l'organe de test à l'intérieur du boîtier.

A partir de cette situation, la présente invention a pour but de proposer un système pour tester un parfum qui soit simple et peu coûteux à fabriquer tout en étant simple et ludique à utiliser.

### -- OBJETS DE L'INVENTION --

A cet effet, l'invention a pour objet un système pour tester un parfum du type précité, caractérisé en ce que le dispositif distributeur de parfum comprend au moins un organe d'imprégnation de parfum avec lequel l'organe de test est adapté pour venir en contact lorsque l'organe de test est inséré à l'intérieur du boîtier, l'organe d'imprégnation de parfum étant adapté pour se charger en parfum et pour imprégner le parfum sur l'organe de test par contact avec l'organe de test.

Grâce à ces dispositions, il est possible de concevoir un système pour tester un parfum de fabrication simple et peu coûteuse et d'utilisation simple et ludique.

Dans divers modes de réalisation du système selon l'invention, on peut avantageusement avoir recours en outre à l'une et/ou à l'autre des dispositions supplémentaires suivantes :
- ledit matériau adapté pour s'imprégner du parfum est un matériau poreux choisi parmi une céramique, le Porex®, une cellulose et un plâtre ;
- l'organe de test comprend une partie de préhension s'étendant selon un axe longitudinal, et une partie d'extrémité disposée dans le prolongement axial de la partie de préhension et faisant saillie radialement de la partie de préhension ;
- l'organe de test comprend une partie de préhension s'étendant selon un axe longitudinal, et une partie d'extrémité disposée dans le prolongement axial de la partie de préhension, la partie d'extrémité étant réalisée à partir dudit matériau adapté pour s'imprégner du parfum et la partie de préhension étant réalisée à partir d'un matériau imperméable au parfum ;
- le dispositif distributeur de parfum comprend une pluralité d'organes d'imprégnation de parfum se présentant sous forme d'éléments parfumés solides ;
- les éléments parfumés sont réalisés sous forme de granulés, de copeaux, de billes ou de capsules ;
- les éléments parfumés sont réalisés à partir de matière plastique, de sels minéraux, de cire, de savon ou de gel ;
- le dispositif distributeur de parfum comprend une recharge de parfum contenant les éléments parfumés ;
- le dispositif distributeur de parfum comprend une recharge de parfum adaptée pour contenir le parfum sous forme liquide et munie d'une lumière obturée par l'organe d'imprégnation de parfum, l'organe d'imprégnation de parfum étant monté à rotation libre sur la recharge de parfum ;
- le dispositif distributeur de parfum comprend une recharge de parfum adaptée pour contenir le parfum sous forme liquide, l'organe d'imprégnation de parfum étant monté fixe sur la recharge de parfum et adapté pour s'imprégner du parfum ;
- le dispositif distributeur de parfum comprend une recharge de parfum adaptée pour contenir le parfum sous forme liquide, et deux organes d'imprégnation de parfum montés à rotation libre sur la recharge de parfum et adaptés pour s'imprégner du parfum ;
- les organes d'imprégnation de parfum sont réalisés à partir de mousse, de Porex®, de cellulose ou de céramique ;
- le système comprend en outre une mèche disposée entre la recharge de parfum et les organes d'imprégnation de parfum, la mèche étant adaptée pour s'imprégner du parfum et pour imprégner le parfum sur les organes d'imprégnation de parfum ; et
- l'organe de test est relié au boîtier par un lien d'attache.

### -- BREVE DESCRIPTION DES DESSINS --

L'invention sera mieux comprise à la lecture de la description qui va suivre de plusieurs modes de réalisation, donnés uniquement à titre d'exemples non limitatifs en se référant aux dessins annexés, sur lesquels :
- les Figures 1 et 2 sont des vues schématiques en perspective d'un système pour tester un parfum selon un premier mode de réalisation de l'invention ;
- la Figure 3 est une vue schématique en perspective d'un système pour tester un parfum selon un deuxième mode de réalisation de l'invention ;
- la Figure 4 est une vue schématique en perspective d'un système pour tester un parfum selon un troisième mode de réalisation de l'invention ;
- les Figures 5A à 5C sont des vues schématiques en coupe verticale longitudinale d'une première variante de réalisation du système de la Figure 4 ;
- la Figure 6 est une vue schématique en coupe verticale longitudinale d'une deuxième variante de réalisation du système de la Figure 4 ; et
- la Figure 7 est une vue schématique en perspective d'un système pour tester un parfum selon un quatrième mode de réalisation de l'invention.

Sur les différentes Figures, les mêmes références désignent des éléments identiques ou similaires.

### -- DESCRIPTION PLUS DETAILLEE --

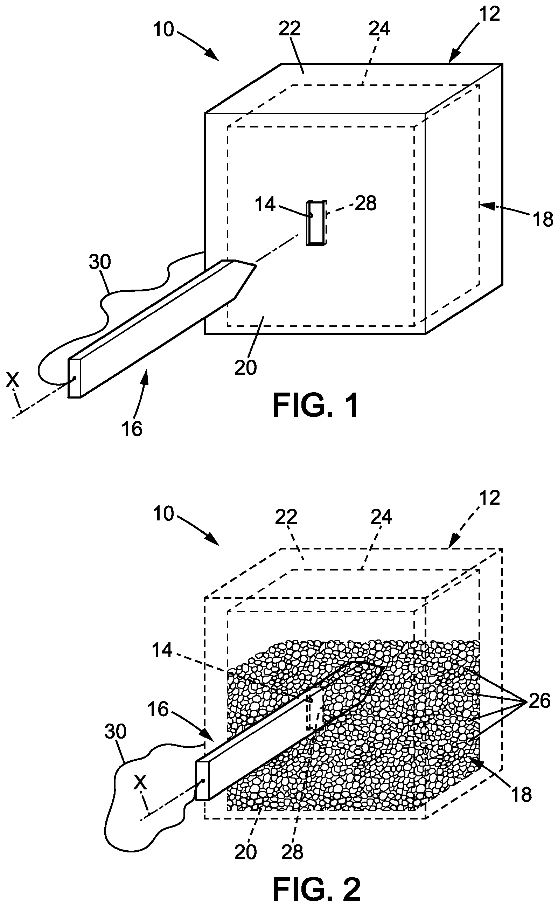

La Figure 1 illustre un système 10 pour tester un parfum selon un premier mode de réalisation de l'invention.

Le système 10 comprend un boîtier 12 comportant une ouverture 14, un organe de test 16 adapté pour être au moins partiellement inséré à l'intérieur du boîtier 12 à travers l'ouverture 14, et un dispositif distributeur 18 de parfum disposé à l'intérieur du boîtier 12 et adapté pour distribuer un parfum sur l'organe de test 16 lorsque l'organe de test 16 est inséré à l'intérieur du boîtier 12.

Dans les différents modes de réalisation considérés, le boîtier 12 se présente sous la forme d'un cube. En variante, le boîtier 12 peut présenter une forme quelconque appropriée, par exemple une forme de pavé, de cylindre, de cône, etc.

Le boîtier 12 peut être réalisé à partir d'un matériau quelconque approprié, par exemple du verre, une matière plastique, etc.

Avantageusement, le boîtier 12 peut être transparent ou translucide, permettant ainsi à un utilisateur de voir le contenu du boîtier 12.

L'ouverture 14 est ménagée sur une des faces latérales 20 du boîtier 12. En variante, l'ouverture 14 peut être ménagée sur la face supérieure 22 du boîtier 12.

Dans les différents modes de réalisation considérés, l'ouverture 14 possède une forme rectangulaire. En variante, l'ouverture 14 peut présenter une forme quelconque appropriée, par exemple carrée, circulaire, triangulaire, etc.

Dans ce premier mode de réalisation, le dispositif distributeur 18 de parfum comprend une recharge 24 de parfum et une pluralité d'organes d'imprégnation 26 de parfum (Figure 2) contenus dans la recharge 24 de parfum.

La recharge 24 de parfum présente une forme identique à celle du boîtier 12 avec des dimensions légèrement inférieures à celles du boîtier 12, ainsi qu'une ouverture 28 placée en correspondance avec l'ouverture 14 du boîtier 12. En variante, la recharge 24 de parfum peut présenter une forme quelconque appropriée, par exemple une forme de pavé, de cylindre, de cône, etc.

Les organes d'imprégnation 26 de parfum sont adaptés pour se charger en parfum et pour imprégner le parfum sur l'organe de test 16 par contact avec l'organe de test 16 lorsque l'organe de test 16 est inséré à l'intérieur du boîtier 12.

En référence à la Figure 2, les organes d'imprégnation 26 de parfum se présentent sous forme d'éléments parfumés solides disposés en vrac à l'intérieur de la recharge 24 de parfum.

De préférence, les éléments parfumés 26 ne remplissent pas complètement la recharge 24 de parfum afin non seulement de faciliter l'insertion et le retrait de l'organe de test 16 dans la recharge 24 de parfum, mais également afin de pouvoir secouer la recharge 24 de parfum pour mélanger les éléments parfumés 26.

En variante, le dispositif distributeur 18 de parfum peut ne pas comprendre de recharge 24 de parfum. Dans ce cas, les éléments parfumés 26 sont disposés en vrac directement à l'intérieur du boîtier 12.

Les éléments parfumés 26 peuvent être réalisés sous une forme quelconque appropriée, par exemple sous forme de granulés, de copeaux, de billes, de capsules, etc.

Les éléments parfumés 26 peuvent être réalisés à partir de :
- matière plastique, par exemple une matière plastique parfumée par imprégnation d'un concentré de parfum (Pebax®, Torrex®, éthylène-acétate de vinyle EVA, etc.) ou une matière plastique parfumée expansée (EVA, etc.) ;
- sels minéraux, par exemple des sels de bain parfumés ;
- cire parfumée ;
- savon ;
- gel parfumé ;
- etc.

Par exemple, les éléments parfumés 26 peuvent être des granulés de Pebax® possédant un diamètre moyen de l'ordre de 3 à 4 mm.

L'organe de test 16 est un organe rigide s'étendant selon un axe longitudinal X et adapté pour être au moins partiellement inséré à l'intérieur du boîtier 12 à travers l'ouverture 14 de manière à venir en contact avec les éléments parfumés 26.

Pour cela, l'organe de test 16 présente une section transversale complémentaire de la forme de l'ouverture 14, à savoir rectangulaire dans l'exemple décrit ici.

L'organe de test 16 est au moins partiellement, totalement dans l'exemple décrit ici, réalisé à partir d'un matériau adapté pour s'imprégner du parfum.

Par exemple, l'organe test 16 peut être fabriqué à partir d'un matériau poreux choisi parmi une céramique, le Porex®, une cellulose, un plâtre, etc., et plus généralement de tout matériau adapté pour s'imprégner de molécules odorantes à l'état liquide ou gazeux.

Afin d'éviter que l'organe de test 16 ne soit volé, perdu ou qu'il ne tombe au sol lorsque le système 10 est disposé en hauteur, l'organe de test 16 est relié au boîtier 12 par un lien d'attache 30, par exemple une cordelette, une chaînette ou encore un lien en cuir.

Le système 10 fonctionne comme suit.

Initialement, l'organe de test 16 est inséré à l'intérieur du boîtier 12 et de la recharge 24 de parfum à travers les ouvertures respectives 14, 28 (Figure 2) et laissé en permanence dans cette position.

L'organe de test 16 est donc en contact avec les éléments parfumés 26 qui imprègnent le parfum sur l'organe de test 16.

Lorsque l'utilisateur souhaite sentir le parfum, il extrait l'organe de test 16 de la recharge 24 de parfum et du boîtier 12 et sent le parfum qui s'est imprégné sur l'organe de test 16.

Lorsqu'il a terminé, l'utilisateur remet l'organe de test 16 dans le boîtier 12 et la recharge 24 de parfum en contact avec les éléments parfumés 26.

Régulièrement, un opérateur peut ouvrir le boîtier 12 et sortir la recharge 24 de parfum pour la secouer, par exemple une fois par jour, de manière à mélanger les éléments parfumés 26 et obtenir une imprégnation optimale du parfum sur l'organe de test 16.

En variante, l'opérateur peut secouer directement le boîtier 12.

Après une certaine période de temps, par exemple une semaine, l'opérateur peut remplacer la recharge 24 de parfum par une recharge de parfum identique ou par une recharge de parfum contenant des éléments parfumés d'un parfum différent.

Lorsque le système 10 ne comprend pas de recharge 24 de parfum, il est bien entendu possible de changer directement les éléments parfumés 26 contenus dans le boîtier 12.

L'organe de test 16 peut également être remplacé par un organe de test identique.

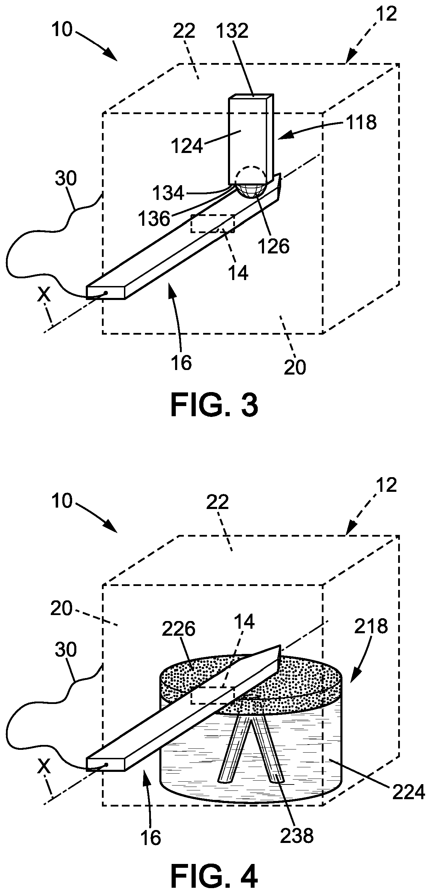

La Figure 3 illustre le système 10 pour tester un parfum selon un deuxième mode de réalisation de l'invention.

Ce deuxième mode de réalisation diffère du premier mode de réalisation des Figures 1 et 2 par le dispositif distributeur 118 de parfum.

Le dispositif distributeur 118 de parfum comprend une recharge 124 de parfum et un organe d'imprégnation 126 de parfum monté à rotation libre sur la recharge 124 de parfum.

La recharge 124 de parfum est adaptée pour contenir le parfum sous forme liquide, par exemple sous forme d'huile ou de solution alcoolique.

La recharge 124 de parfum présente par exemple une forme de pavé. En variante, la recharge 124 de parfum peut présenter une forme quelconque appropriée, par exemple de cube, de cylindre, etc.

La recharge 124 de parfum est fixée par sa face supérieure 132 sur la surface interne de la face supérieure 22 du boîtier 12.

La face inférieure 134 de la recharge 124 de parfum est munie d'une lumière 136 obturée par l'organe d'imprégnation 126 de parfum.

Dans l'exemple considéré, la lumière 136 est circulaire et l'organe d'imprégnation 126 de parfum est formé par une bille.

Le système 10 fonctionne comme suit.

Initialement, l'organe de test 16 est inséré dans le boîtier 12 à travers l'ouverture 14 et laissé en permanence en contact avec la bille 126.

Lorsque l'utilisateur souhaite sentir le parfum, il extrait l'organe de test 16 du boîtier 12, ce qui fait rouler la bille 126 sur elle-même. La bille 126 se charge alors en parfum, par exemple à sa surface, et dépose ainsi le parfum sur l'organe de test 16.

L'utilisateur sent alors le parfum imprégné sur l'organe de test 16 puis, lorsqu'il a terminé, il remet l'organe de test 16 dans le boîtier 12 en contact avec la bille 126.

Lorsque la recharge 124 de parfum est vide, l'opérateur peut ouvrir le boîtier 12 et remplacer le dispositif distributeur 118 de parfum par un dispositif distributeur de parfum identique ou par un dispositif distributeur de parfum dans lequel la recharge de parfum contient un parfum différent.

Un avantage procuré par ce deuxième mode de réalisation réside dans le fait qu'il permet à l'utilisateur d'avoir les notes de fond, les notes de cœur et les notes de tête du parfum. En effet, les notes de fond et les notes de cœur sont obtenues grâce aux insertions/extractions précédentes de l'organe de test tandis que les notes de tête sont obtenues grâce à la dernière extraction de l'organe de test.

La Figure 4 illustre le système 10 pour tester un parfum selon un troisième mode de réalisation de l'invention.

Ce troisième mode de réalisation diffère du deuxième mode de réalisation de la Figure 3 par le dispositif distributeur 218 de parfum.

Le dispositif distributeur 218 de parfum comprend une recharge 224 de parfum et un organe d'imprégnation 226 de parfum.

La recharge 224 de parfum est adaptée pour contenir le parfum sous forme liquide, par exemple sous forme d'huile ou de solution alcoolique.

La recharge 224 de parfum est disposée au fond du boîtier 12 et présente par exemple une forme cylindrique. En variante, la recharge 224 de parfum peut présenter une forme quelconque appropriée, par exemple une forme de cube, de pavé, etc.

L'organe d'imprégnation 226 de parfum est monté fixe sur la recharge 224 de parfum et est adapté pour s'imprégner du parfum contenu dans la recharge 224 de parfum.

Pour cela, l'organe d'imprégnation 226 de parfum peut être réalisé par exemple à partir de mousse, de Porex®, de cellulose, de céramique, etc., et plus généralement de tout matériau perméable à des molécules olfactives liquides.

Avantageusement, une mèche 238 peut être prévue entre la recharge 224 de parfum et l'organe d'imprégnation 226 de parfum pour s'imprégner du parfum contenu dans la recharge 224 de parfum et pour imprégner le parfum sur l'organe d'imprégnation 226 de parfum.

Le système 10 fonctionne comme suit.

Initialement, l'organe de test 16 est inséré dans le boîtier 12 à travers l'ouverture 14 et laissé en permanence en contact avec l'organe d'imprégnation 226 de parfum qui imprègne le parfum sur l'organe de test 16.

Lorsque l'utilisateur souhaite sentir le parfum, il extrait l'organe de test 16 du boîtier 12, ce qui imprègne de nouveau le parfum sur l'organe de test 16.

L'utilisateur sent alors le parfum imprégné sur l'organe de test 16 puis remet l'organe de test 16 dans le boîtier 12 en contact avec l'organe d'imprégnation 226 de parfum.

Lorsque la recharge 224 de parfum est vide, l'opérateur peut ouvrir le boîtier 12 et remplacer le dispositif distributeur 218 de parfum par un dispositif distributeur de parfum identique ou par un dispositif distributeur de parfum dans lequel la recharge de parfum contient un parfum différent.

Ce troisième mode de réalisation permet également à l'utilisateur d'obtenir les notes de fond, de cœur et de tête du parfum.

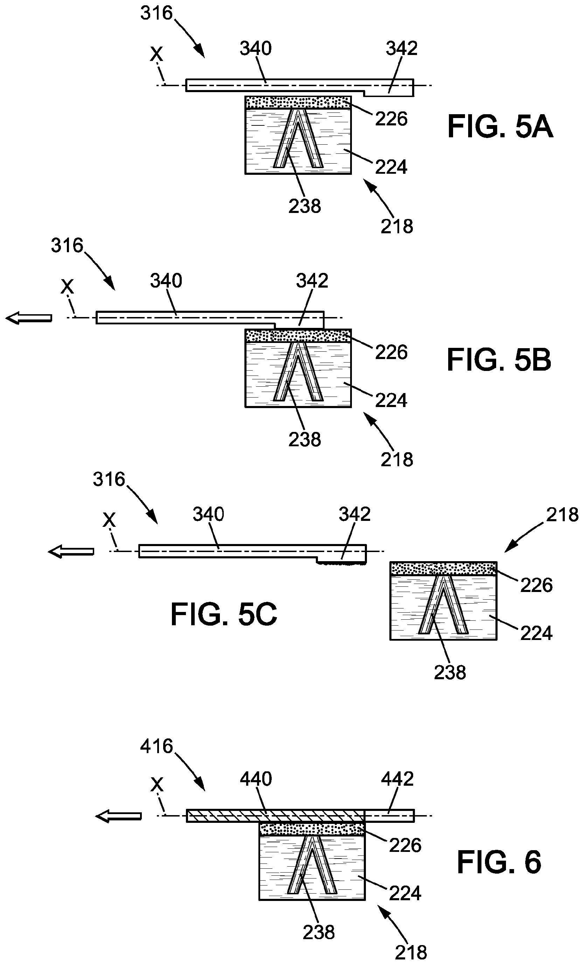

Une première variante de ce troisième mode de réalisation est illustrée sur les Figures 5A à 5C.

Cette première variante diffère du troisième mode de réalisation de la Figure 4 par l'organe de test 316 qui comprend une partie de préhension 340 s'étendant selon l'axe longitudinal X et une partie d'extrémité 342 disposée dans le prolongement axial de la partie de préhension 340 et faisant saillie radialement de la partie de préhension 340.

Le système 10 fonctionne comme suit.

Initialement, l'organe de test 316 est inséré dans le boîtier 12 à travers l'ouverture 14 et laissé en permanence dans une position telle que la partie de préhension 340 se trouve en regard de l'organe d'imprégnation 226 de parfum mais pas en contact avec l'organe d'imprégnation 226 de parfum qui est chargé en parfum (Figure 5A).

Lorsque l'utilisateur souhaite sentir le parfum, il extrait l'organe de test 316 du boîtier 12 (Figure 5B).

Lors de cette extraction, la partie d'extrémité 342 vient en contact avec l'organe d'imprégnation 226 de parfum et s'imprègne ainsi du parfum (Figure 5C).

L'utilisateur sent ensuite le parfum imprégné sur la partie d'extrémité 342 puis remet l'organe de test 316 dans le boîtier 12, la partie de préhension 340 se retrouvant en regard mais pas en contact avec l'organe d'imprégnation 226 de parfum.

Au bout d'une semaine par exemple, l'organe de test 316 peut être remplacé par un organe de test identique ou par l'organe de test 16 des Figures 1 à 4.

Cette variante de réalisation permet également à l'utilisateur d'obtenir les notes de fond, de cœur et de tête du parfum.

Un autre avantage procuré par cette variante de réalisation réside dans le fait que, lorsque le système n'est pas utilisé, l'organe de test ne s'imprègne pas du parfum inutilement et la recharge de parfum ne se vide pas non plus du parfum inutilement. Le système présente ainsi une durée de vie prolongée.

Dans une deuxième variante de ce troisième mode de réalisation, qui permet également à l'utilisateur d'avoir les notes de fond, de cœur et de tête du parfum et qui permet aussi de prolonger la durée de vie du système, l'organe de test 316 peut être remplacée par l'organe de test 416 de la Figure 6.

L'organe de test 416 comporte une partie de préhension 440 s'étendant selon l'axe longitudinal X et une partie d'extrémité 442 disposée dans le prolongement axial de la partie de préhension 440.

La partie d'extrémité 442 est réalisée à partir d'un matériau adapté pour s'imprégner du parfum, de manière identique aux autres modes de réalisation, mais la partie de préhension 440 est réalisée à partir d'un matériau imperméable au parfum, en particulier un matériau non poreux.

Le système 10 fonctionne comme suit.

Initialement, l'organe de test 416 est inséré dans le boîtier 12 à travers l'ouverture 14 et laissé en permanence dans une position telle que la partie de préhension 440 se trouve en regard de l'organe d'imprégnation 226 de parfum et en contact avec l'organe d'imprégnation 226 de parfum qui est chargé en parfum.

La partie de préhension 440 étant imperméable au parfum, elle ne s'imprègne pas du parfum.

Lorsque l'utilisateur souhaite sentir le parfum, il extrait l'organe de test 416 du boîtier 12.

Lors de cette extraction, la partie d'extrémité 442 vient en contact avec l'organe d'imprégnation 226 de parfum et s'imprègne ainsi du parfum.

L'utilisateur sent ensuite le parfum imprégné sur la partie d'extrémité 442 puis remet l'organe de test 416 dans le boîtier 12, la partie de préhension 440 se retrouvant en regard et en contact avec l'organe d'imprégnation 226 de parfum.

Au bout d'une semaine par exemple, l'organe de test 416 peut être remplacé par un organe de test identique ou par les organes de test 16, 316 des Figures 1 à 5.

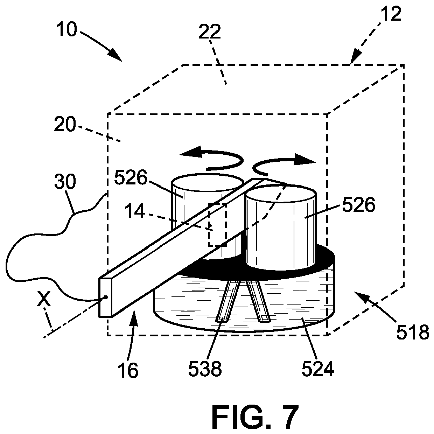

La Figure 7 illustre le système 10 pour tester un parfum selon un quatrième mode de réalisation de l'invention.

Ce quatrième mode de réalisation diffère du troisième mode de réalisation de la Figure 4 par le dispositif distributeur 518 de parfum.

Le dispositif distributeur 518 de parfum comprend une recharge 524 de parfum et deux organes d'imprégnation 526 de parfum.

La recharge 524 de parfum est adaptée pour contenir le parfum sous forme liquide, par exemple sous forme d'huile ou de solution alcoolique.

La recharge 524 de parfum est disposée au fond du boîtier 12 et présente par exemple une forme cylindrique. En variante, la recharge 524 de parfum peut présenter une forme quelconque appropriée, par exemple une forme de cube, de pavé, etc.

Les organes d'imprégnation 526 de parfum sont montés à rotation libre sur la recharge 524 de parfum et sont adaptés pour s'imprégner du parfum contenu dans la recharge 524 de parfum.

Pour cela, les organes d'imprégnation 226 de parfum peuvent être réalisés par exemple à partir de mousse, de Porex®, de cellulose, de céramique, etc., et plus généralement de tout matériau perméable à des molécules olfactives liquides.

Avantageusement, une mèche 538 peut être prévue entre la recharge 524 de parfum et l'organe d'imprégnation 526 de parfum pour faciliter l'imprégnation du parfum sur l'organe d'imprégnation 526 de parfum.

Le système 10 fonctionne comme suit.

Initialement, l'organe de test 16 est inséré dans le boîtier 12 à travers l'ouverture 14 et laissé en permanence en contact avec et entre les organes d'imprégnation 526 de parfum chargés en parfum qui imprègnent le parfum sur l'organe de test 16.

Lorsque l'utilisateur souhaite sentir le parfum, il extrait l'organe de test 16 du boîtier 12, ce qui fait tourner les organes d'imprégnation 526 de parfum sur eux-mêmes dans des sens de rotation opposés et imprègne de nouveau le parfum sur l'organe de test 16.

L'utilisateur sent alors le parfum imprégné sur l'organe de test 16 puis remet l'organe de test 16 dans le boîtier 12 en contact avec les organes d'imprégnation 226 de parfum.

Lorsque la recharge 524 de parfum est vide, l'opérateur peut ouvrir le boîtier 12 et remplacer le dispositif distributeur 518 de parfum par un dispositif distributeur de parfum identique ou par un dispositif distributeur de parfum dans lequel la recharge de parfum contient un parfum différent.

Ce quatrième mode de réalisation permet également à l'utilisateur d'obtenir les notes de fond, de cœur et de tête du parfum.

L'invention propose donc un système pour tester un parfum qui est simple et peu coûteux à fabriquer, et simple et ludique à utiliser.

De plus, la distribution du parfum sur l'organe de test étant réalisée par contact avec l'organe d'imprégnation de parfum et non par pulvérisation, le système selon l'invention permet d'éviter que des gouttes de parfum ne soient projetées ailleurs que sur l'organe de test, limitant ainsi le gaspillage du parfum et la salissure du boîtier.

En outre, le fait de disposer d'une recharge de parfum permet de faciliter le remplacement du dispositif distributeur de parfum lorsqu'il n'y a plus de parfum ou lorsque l'opérateur souhaite changer le parfum à tester.

Le système selon l'invention permet également de laisser l'organe de test à l'intérieur du boîtier de façon prolongée, par exemple toute une nuit, permettant ainsi à l'utilisateur d'accéder aux notes de fond et aux notes de cœur du parfum en plus des notes de tête qui sont généralement les seules obtenues avec des testeurs classiques.

Le système selon l'invention peut en outre être esthétique, avec par exemple un boîtier transparent ou translucide.

## Revendications

1. Système (10) pour tester un parfum comprenant :
- un boîtier (12) comportant une ouverture (14) ;
- un organe de test (16, 316, 416) adapté pour être au moins partiellement inséré à l'intérieur du boîtier à travers l'ouverture, l'organe de test étant réalisé au moins partiellement à partir d'un matériau adapté pour s'imprégner du parfum ; et
- un dispositif distributeur (18, 118, 218, 518) de parfum disposé à l'intérieur du boîtier et adapté pour distribuer le parfum sur l'organe de test lorsque l'organe de test est inséré à l'intérieur du boîtier,
**caractérisé en ce que** le dispositif distributeur de parfum comprend au moins un organe d'imprégnation (26, 126, 226, 526) de parfum avec lequel l'organe de test est adapté pour venir en contact lorsque l'organe de test est inséré à l'intérieur du boîtier, l'organe d'imprégnation de parfum étant adapté pour se charger en parfum et pour imprégner le parfum sur l'organe de test par contact avec l'organe de test.

2. Système (10) selon la revendication 1, dans lequel ledit matériau adapté pour s'imprégner du parfum est un matériau poreux choisi parmi une céramique, le Porex®, une cellulose et un plâtre.

3. Système (10) selon la revendication 1 ou 2, dans lequel l'organe de test (316) comprend une partie de préhension (340) s'étendant selon un axe longitudinal (X), et une partie d'extrémité (342) disposée dans le prolongement axial de la partie de préhension et faisant saillie radialement de la partie de préhension.

4. Système (10) selon la revendication 1 ou 2, dans lequel l'organe de test (416) comprend une partie de préhension (440) s'étendant selon un axe longitudinal (X), et une partie d'extrémité (442) disposée dans le prolongement axial de la partie de préhension, la partie d'extrémité étant réalisée à partir dudit matériau adapté pour s'imprégner du parfum et la partie de préhension étant réalisée à partir d'un matériau imperméable au parfum.

5. Système (10) selon l'une quelconque des revendications 1 à 4, dans lequel le dispositif distributeur (18) de parfum comprend une pluralité d'organes d'imprégnation de parfum se présentant sous forme d'éléments parfumés (26) solides.

6. Système (10) selon la revendication 5, dans lequel les éléments parfumés (26) sont réalisés sous forme de granulés, de copeaux, de billes ou de capsules.

7. Système (10) selon la revendication 5 ou 6, dans lequel les éléments parfumés (26) sont réalisés à partir de matière plastique, de sels minéraux, de cire, de savon ou de gel.

8. Système (10) selon l'une quelconque des revendications 5 à 7, dans lequel le dispositif distributeur (18) de parfum comprend une recharge (24) de parfum contenant les éléments parfumés (26).

9. Système (10) selon l'une quelconque des revendications 1 à 4, dans lequel le dispositif distributeur (118) de parfum comprend une recharge (124) de parfum adaptée pour contenir le parfum sous forme liquide et munie d'une lumière (136) obturée par l'organe d'imprégnation (126) de parfum, l'organe d'imprégnation de parfum étant monté à rotation libre sur la recharge de parfum.

10. Système (10) selon l'une quelconque des revendications 1 à 4, dans lequel le dispositif distributeur (218) de parfum comprend une recharge (224) de parfum adaptée pour contenir le parfum sous forme liquide, l'organe d'imprégnation (226) de parfum étant monté fixe sur la recharge de parfum et adapté pour s'imprégner du parfum.

11. Système (10) selon l'une quelconque des revendications 1 à 4, dans lequel le dispositif distributeur (518) de parfum comprend une recharge (524) de parfum adaptée pour contenir le parfum sous forme liquide, et deux organes d'imprégnation (526) de parfum montés à rotation libre sur la recharge de parfum et adaptés pour s'imprégner du parfum.

12. Système (10) selon la revendication 10 ou 11, dans lequel les organes d'imprégnation (226, 526) de parfum sont réalisés à partir de mousse, de Porex®, de cellulose ou de céramique.

13. Système (10) selon l'une quelconque des revendications 10 à 12, comprenant en outre une mèche (238, 538) disposée entre la recharge (224, 524) de parfum et les organes d'imprégnation (226, 526) de parfum, la mèche étant adaptée pour s'imprégner du parfum et pour imprégner le parfum sur les organes d'imprégnation de parfum.

14. Système (10) selon l'une quelconque des revendications 1 à 13, dans lequel l'organe de test (16, 316, 416) est relié au boîtier (12) par un lien d'attache (30).

## Patentansprüche

1. System (10) zum Testen eines Parfums aufweisend:
- ein Gehäuse (12) mit einer Öffnung (14);
- ein Testelement (16, 316, 416), das eingerichtet ist, über die Öffnung zumindest teilweise in das Innere des Gehäuses eingeführt zu werden, wobei das Testelement zumindest teilweise aus einem Material hergestellt ist, das eingerichtet ist, sich mit Parfum zu tränken; und
- eine Parfumabgabevorrichtung (18, 118, 218, 518), die im Inneren des Gehäuses angeordnet ist und eingerichtet ist, das Parfum auf das Testelement abzugeben, wenn das Testelement in das Innere des Gehäuses eingeführt ist,
**dadurch gekennzeichnet, dass** die Parfumabgabevorrichtung mindestens ein Parfumtränkungselement (26, 126, 226, 526) aufweist, mit dem das Testelement in Kontakt kommen kann, wenn das Testelement in das Innere des Gehäuses eingeführt ist, wobei das Parfumtränkungselement eingerichtet ist, sich mit Parfum zu beladen und durch Kontakt mit dem Testelement das Testelement mit dem Parfum zu tränken.

2. System (10) nach Anspruch 1, in welchem das Material, das eingerichtet ist, sich mit Parfum zu tränken, ein poröses Material ist, das aus einer Keramik, Porex®, einer Zellulose und einem Gips ausgewählt ist.

3. System (10) nach Anspruch 1 oder 2, in welchem das Testelement (316) einen sich entlang einer Längsachse (X) erstreckenden Greifabschnitt (340) und einen in der axialen Verlängerung des Greifabschnitts angeordneten und von dem Greifabschnitt radial vorspringenden Endabschnitt (342) aufweist.

4. System (10) nach Anspruch 1 oder 2, in welchem das Testelement (416) einen sich entlang einer Längsachse (X) erstreckenden Greifabschnitt (440) und einen in der axialen Verlängerung des Greifabschnitts angeordneten Endabschnitt (442) aufweist, wobei der Endabschnitt aus dem Material, das eingerichtet ist, sich mit Parfum zu tränken, hergestellt ist und der Greifabschnitt aus einem für das Parfum undurchdringbaren Material hergestellt ist.

5. System (10) nach einem der Ansprüche 1 bis 4, in welchem die Parfumabgabevorrichtung (18) mehrere Parfumtränkungselemente aufweist, die in Form von parfümierten Feststoffelementen (26) vorliegen.

6. System (10) nach Anspruch 5, in welchem die parfümierten Elemente (26) in Form von Granulat, Spänen, Kugeln oder Kapseln ausgeführt sind.

7. System (10) nach Anspruch 5 oder 6, in welchem die parfümierten Elemente (26) aus einem Kunststoffmaterial, Mineralsalzen, Wachs, Seife oder Gel hergestellt sind.

8. System (10) nach einem der Ansprüche 5 bis 7, in welchem die Parfumabgabevorrichtung (18) eine Parfumnachfüllpackung (24) aufweist, die die parfümierten Elemente (26) enthält.

9. System (10) nach einem der Ansprüche 1 bis 4, in welchem die Parfumabgabevorrichtung (118) eine Parfumnachfüllpackung (124) aufweist, die eingerichtet ist, das Parfum in flüssiger Form zu enthalten, und mit einem von dem Parfumtränkungselement (126) verschlossenen Loch (126) versehen ist, wobei das Parfumtränkungselement frei rotierbar an der Parfumnachfüllpackung angebracht ist.

10. System (10) nach einem der Ansprüche 1 bis 4, in welchem die Parfumabgabevorrichtung (218) eine Parfumnachfüllpackung (224) aufweist, die eingerichtet ist, das Parfum in flüssiger Form zu enthalten, wobei das Parfumtränkungselement (226) fest an der Parfumnachfüllpackung angebracht ist und eingerichtet ist, sich mit Parfum zu tränken.

11. System (10) nach einem der Ansprüche 1 bis 4, in welchem die Parfumabgabevorrichtung (518) aufweist: eine Parfumnachfüllpackung (524), die eingerichtet ist, das Parfum in flüssiger Form zu enthalten, und zwei Parfumtränkungselemente (526), die frei rotierbar an der Parfumnachfüllpackung angebracht sind und eingerichtet sind, sich mit Parfum zu tränken.

12. System (10) nach Anspruch 10 oder 11, in welchem die Parfumtränkungselemente (226, 526) aus Schaum, Porex®, Zellulose oder Keramik hergestellt sind.

13. System (10) nach einem der Ansprüche 10 bis 12, ferner aufweisend einen Docht (238, 538), der zwischen der Parfumnachfüllpackung (224, 524) und den Parfumtränkungselementen (226, 526) angeordnet ist, wobei der Docht eingerichtet ist, sich mit Parfum zu tränken und die Parfumtränkungselemente mit dem Parfum zu tränken.

14. System (10) nach einem der Ansprüche 1 bis 13, in welchem das Testelement (16, 316, 416) über ein Befestigungsband (30) mit dem Gehäuse (12) verbunden ist.

## Claims

1. System (10) for testing a perfume, comprising:
- a housing (12) comprising an opening (14);
- a test body (16, 316, 416) designed to be at least partially inserted into the housing through the opening, the test body being made at least partially from a material that can be impregnated with perfume; and
- a perfume-dispensing device (18, 118, 218, 518) arranged inside the housing and designed to dispense the perfume onto the test body when the test body is inserted into the housing,
**characterized in that** the perfume-dispensing device comprises at least one perfume-impregnating body (26, 126, 226, 526) with which the test body is designed to come into contact when the test body is inserted into the housing, the perfume-impregnating body being designed to be loaded with perfume and to impregnate the test body with the perfume when it comes into contact with the test body.

2. System (10) according to claim 1, wherein said material that can be impregnated with perfume is a porous material selected from among a ceramic, Porex®, a cellulose, and a plaster.

3. System (10) according to claim 1 or 2, wherein the test body (316) comprises a gripping portion (340) extending along a longitudinal axis (X), and an end portion (342) arranged in the axial extension of the gripping portion and projecting radially from the gripping portion.

4. System (10) according to claim 1 or 2, wherein the test body (416) comprises a gripping portion (440) extending along a longitudinal axis (X), and an end portion (442) arranged in the axial extension of the gripping portion, the end portion being made of said material that can be impregnated with perfume and the gripping portion being made of a material impermeable to perfume.

5. System (10) according to any one of claims 1 to 4, wherein the perfume-dispensing device (18) comprises a plurality of perfume-impregnating bodies in the form of solid perfumed elements (26).

6. System (10) according to claim 5, wherein the perfumed elements (26) are in the form of granules, chips, beads, or capsules.

7. System (10) according to claim 5 or 6, wherein the perfumed elements (26) are made from plastic, mineral salts, wax, soap, or gel.

8. System (10) according to any one of claims 5 to 7, wherein the perfume-dispensing device (18) comprises a perfume charger (24) containing the perfumed elements (26).

9. System (10) according to any one of claims 1 to 4, wherein the perfume-dispensing device (118) comprises a perfume charger (124) designed to contain perfume in liquid form and provided with a hole (136) sealed by the perfume-impregnating body (126), the perfume-impregnating body being mounted to rotate freely on the perfume charger.

10. System (10) according to any one of claims 1 to 4, wherein the perfume-dispensing device (218) comprises a perfume charger (224) designed to contain perfume in liquid form, the perfume-impregnating body (226) being fixedly mounted on the perfume charger and designed to be impregnated with perfume.

11. System (10) according to any one of claims 1 to 4, wherein the perfume-dispensing device (518) comprises a perfume charger (524) designed to contain perfume in liquid form, and two perfume-impregnating bodies (526) mounted to rotate freely on the perfume charger and designed to be impregnated with perfume.

12. System (10) according to claim 10 or 11, wherein the perfume-impregnating bodies (226, 526) are made from foam, Porex®, cellulose, or ceramic.

13. System (10) according to any one of claims 10 to 12, further comprising a wick (238, 538) arranged between the perfume charger (224, 524) and the perfume-impregnating bodies (226, 526), the wick being designed to soak up the perfume and to impregnate the perfume-impregnating bodies with perfume.

14. System (10) according to any one of claims 1 to 13, wherein the test body (16, 316, 416) is connected to the housing (12) by a tether (30).
